# EUROPEAN PATENT SPECIFICATION

(11) **EP 3 526 347 B1**
(45) Date of publication and mention of the grant of the patent: **13.01.2021**
(21) Application number: 17791982.6
(22) Date of filing: 13.10.2017
(51) Int. Cl.: C12Q 1/68, C12Q 1/6886

(54) **INDOLEAMINE-2,3-DIOXYGENASE ASSAY FOR PROSTATE CANCER DIAGNOSIS AND PROGNOSIS**
INDOLEAMIN-2,3-DIOXYGENAS-ASSAY ZUR PROSTATAKREBSDIAGNOSE UND -PROGNOSE
ANALYSE D'INDOLÉAMINE-2,3-DIOXYGÉNASE POUR LE DIAGNOSTIC ET PROGNOSTIC DU CANCER DE LA PROSTATE

(30) Priority: 14.10.2016 EP 16194043; 30.06.2017 EP 17179152
(43) Date of publication of application: 21.08.2019
(73) Proprietor: Universität Zürich, 8006 Zürich (CH)
(72) Inventor: BANZOLA, Irina, 8052 Zürich (CH); PROVENZANO, Maurizio, 5708 Birrwil (AG) (CH)
(74) Representative: Schulz Junghans Patentanwälte PartGmbB
(86) International application number: PCT/EP2017/076169
(87) International publication number: WO 2018/069494

(56) References cited:
- WO-A1-2013/028788
- WO-A1-2013/190127
- WO-A1-2015/077414
- FEDER-MENGUS C ET AL: "High expression of indoleamine 2,3-dioxygenase gene in prostate cancer", EUROPEAN JOURNAL OF CANCER, ELSEVIER, AMSTERDAM, NL, vol. 44, no. 15, 1 October 2008 (2008-10-01), pages 2266-2275, XP025608945, ISSN: 0959-8049, DOI: 10.1016/J.EJCA.2008.05.023 [retrieved on 2008-07-09]
- ZHAI LIJIE ET AL: "The role of IDO in brain tumor immunotherapy", JOURNAL OF NEURO-ONCOLOGY, KLUWER, BOSTON, US, vol. 123, no. 3, 18 December 2014 (2014-12-18), pages 395-403, XP035510777, ISSN: 0167-594X, DOI: 10.1007/S11060-014-1687-8 [retrieved on 2014-12-18]
- VLAEMINCK-GUILLEM V ET AL: "Urinary Prostate Cancer 3 Test: Toward the Age of Reason?", UROLOGY, BELLE MEAD, NJ, US, vol. 75, no. 2, 1 February 2010 (2010-02-01), pages 447-453, XP026885615, ISSN: 0090-4295, DOI: 10.1016/J.UROLOGY.2009.03.046 [retrieved on 2009-07-08]
- AMALIA KATAFIGIOTI ET AL: "In the search of novel urine biomarkers for the early diagnosis of prostate cancer. Intracellular or secreted proteins as the target group? Where and how to search for possible biomarkers useful in the everyday clinical practice", ARCHIVIO ITALIANO DI UROLOGIA, ANDROLOGIA : UROLOGICAL AND ANDROLOGICAL SCIENCES ; ORGANO UFFICIALE, vol. 88, no. 3, 5 October 2016 (2016-10-05), page 195, XP055440884, IT ISSN: 1124-3562, DOI: 10.4081/aiua.2016.3.195
- EIHAB AL KHASAWNEH ET AL: "Stable pediatric kidney transplant recipients run higher urine indoleamine 2, 3 dioxygenase (IDO) levels than healthy children", PEDIATRIC TRANSPLANTATION, vol. 18, no. 3, 1 February 2014 (2014-02-01), pages 254-257, XP055440899, DK ISSN: 1397-3142, DOI: 10.1111/petr.12232

### SEQUENCE LISTING

<110> Universitaet Zuerich
<120> Indoleamine-2,3-dioxygenase assay for prostate cancer diagnosis and prognosis
<130> uz266wo
<160> 3
<170> PatentIn version 3.5
<210> 1
   <211> 20
   <212> DNA
   <213> homo sapiens
<400> 1
   gaagacccaa aggagtttgc 20
<210> 2
   <211> 19
   <212> DNA
   <213> homo sapiens
<400> 2
   tggaggaact gagcagcat 19
<210> 3
   <211> 17
   <212> DNA
   <213> homo sapiens
<400> 3
   tgggcatcca gcagact 17

## Description

### Background:

Prostate cancer (PCa) is the most common cancer in men, and a leading cause of male cancer mortality in developed countries. Most cases of terminal prostate cancer originate through a process of progression from a slow-growing, organ-confined tumor to a highly invasive castration-resistant prostate cancer (CRPC).

According to a large study (Schroder et al., (2009), New England J Med 360, 1320-8), current prostate specific antigen (PSA) based screening reduces the rate of death from prostate cancer by 20% but is associated with a high risk of overdiagnosis, Rev Urol. 2009;11(3):127-133 doi: 10.3909/riu0474 .

Currently, prostate biopsy is the golden standard procedure for prostate cancer detection, despite its association with potential harmful side effects for patients, its high costs and a definite rate of false positive (11.5% as reported by Epstein et al, Utility of saturation biopsy to predict insignificant cancer at radical prostatectomy, volume 66, Issue 2 August 2005 Pages 356-360) and negative (23% according to Rabbani et al., Incidence and clinical significance of false-negative sextant prostate biopsies, The Journal of Urology, Volume 159, Issue 4 72 April 1998, Pages 1247-1250).

Clinically localized prostate cancer is typically managed by well-established therapies like radical prostatectomy, brachytherapy, and external beam radiation therapy. While many patients can be cured with definitive local therapy, some will have biochemical recurrence (BR) of disease detected by a rising serum PSA after treatment with radical prostatectomy.

The US Food and Drug Administration (FDA) has approved a prostate cancer test based on detection of prostate cancer gene 3 (PCA3) in urine, but its clinical utility has been reported to be only marginally better than that of PSA detection (Vlaeminck-Guillem et al., Urology. 2010;75(2):447); Roobol et al., Eur Urol. 2010;58(4):475) Also WO 2013/028788 discloses methods for the diagnosis and prognosis of prostate cancer using urine biomarkers.

Improving current methods for screening of patients is therefore of great importance.

A significant body of literature suggests that inflammation might play an important role in the onset and progression of PCa, although a direct and causal correlation between inflammation and PCa has not been established yet. Considerable epidemiological evidence indicates that PCa is more predominant in demographic groups with a higher degree of inflammation, and that patients affected by obesity show an increased risk of prostate cancer-specific mortality. Furthermore, analysis of prostatic tissues has revealed that in most cases, adenocarcinomas are found adjacent to chronic inflammation.

Upon transformation due to accumulation of several genetic mutations and epigenetic alterations, tumor cells are either fully eliminated or progress to overt cancer, depending on immunity fitness. In particular, tumor immune escape in PCa occurs through the secretion of different tumor-derived soluble factors (TDSFs) with immune suppressive properties, such as indoleamine 2,3-dioxygenase (IDO), arginase, nitric oxide synthase (NOS) and cytokines relevant in coordinating cancer immune-editing and in modulating the TDSF network. Among several micro-environmental modifiers, our interest focused on those that have been reported as possible mediators of PCa onset and progression (such as IDO, IL-6, and IL-1β). Controversial data about the role of the microenvironment in cancer are reported in literature, where the expression of putative tumor-promoting agents only at times correlates with cancer aggressiveness and poor clinical outcome. Indeed, high levels of IDO were correlated with poor clinical prognosis in ovarian, endometrial and colon carcinomas, malignant melanoma, breast and lung cancer but not in renal cell carcinoma (RCC). In this latter case, high levels of IDO mRNA in primary tumors or metastases positively correlated with longer overall survival. The expression of IDO was detected in endothelial cells of newly formed vessels inside the tumor area, not in tumor cells. Other authors have confirmed these data and have hypothesized that IDO produced by endothelial cells would deprive cancer cells of the essential amino acid tryptophan and therefore inhibit their growth.

### Description

The invention provides a method to
- assign a patient to prostate biopsy,
- predict a patient's risk of having or developing prostate cancer,
- predict a patient's risk of having clinically relevant prostate cancer,
- predict a patient's risk of having indolent prostate cancer,
- distinguish indolent vs. aggressive cancers,
- predict a patient's risk of relapse of prostate cancer,
- predict a patient's risk of progression of an indolent prostate cancer to symptomatic prostate cancer.

In any case, the method comprises detecting / quantifying the level of indoleamine-2,3-dioxygenase (IDO) mRNA or protein in a urine sample obtained from the patient.

In certain embodiments, the invention provides a method to
- predict a patient's risk of developing prostate cancer,
- distinguish indolent vs. aggressive cancers among patients indicated to undergo prostate biopsy,
- predict the likelihood of biochemical recurrence of prostate cancer in a patient having undergone prostate cancer treatment, and/or
- predict the risk of progression of said prostate cancer in a patient having undergone prostate cancer treatment, particularly radical prostatectomy, brachytherapy, and/or external beam radiation therapy;

In certain embodiments, the invention provides a method to
- predict a patient's risk of having prostate cancer,
- predict a patient's risk of having clinically relevant prostate cancer with a Gleason score ≥ 7,
- predict a patient's risk of having prostate cancer and/or chronic multifocal prostate inflammation,
- predict a patient's risk of developing prostate cancer,
- assign a patient to prostate biopsy,
- distinguish indolent vs. aggressive cancers among patients indicated to undergo prostate biopsy,
- predict a patient's risk of biochemical recurrence of prostate cancer, and/or
- predict a patient's risk of progression of an indolent prostate cancer to symptomatic prostate cancer.

The inventors have quantified the level of indoleamine-2,3-dioxygenase (IDO) mRNA and protein in urine samples and have determined relative cut-offs that predict whether the patient will benefit from undergoing a prostate biopsy and has a higher than average risk (weighed to the relevant patient group) to progress to biochemical recurrence and/or untreatable disease.

Patients may be selected as at risk of having a prostate tumour by their physician according to the international guidelines (Pre-biopsy nomogram: race, age, family history, PSA level >4ng/ml, DRE outcome, previous biopsies). Patients that are selected as at risk of having a prostate tumour by their physician are indicated to undergo a prostate biopsy. The test according to the invention will select which of these patients must undergo biopsy and which not.

The test according to the invention can be repeatedly performed to monitor the development of PCa, at any time point before or after treatment. Once a positive biopsy is detected and the application of a treatment is decided, the diagnosis is based on this result.

If, after the biopsy, the patient is assigned to active surveillance, the IDO testing can be used to monitor the changes in the development of the lesion. If, after the biopsy, the patient is assigned to treatment, the IDO testing can be used to monitor the presence of residual tumor tissue in the prostate.

In certain embodiments, a patient's risk of progression of an indolent prostate cancer to symptomatic or clinically relevant prostate cancer is determined in a patient selected to perform a biopsy of the prostate.

In certain embodiments, a patient's risk of progression of prostate cancer, particularly the risk of progression of an indolent prostate cancer to symptomatic or clinically relevant prostate cancer is determined in a patient having undergone prostate cancer treatment.

In certain embodiments, a patient's risk of biochemical recurrence of prostate cancer is determined.

In certain embodiments, a patient's risk of biochemical recurrence of prostate cancer is determined in a patient having undergone prostate cancer treatment. In certain embodiments, the prostate cancer treatment is selected from radical prostatectomy, brachytherapy, and/or external beam radiation therapy.ln certain embodiments, a patient's risk of relapse of prostate cancer is determined.

In certain embodiments, no DRE is performed prior to obtaining the urine sample. The method according to the invention yields significant results without execution of a DRE. The abandonment of a DRE increases patient compliance and renders the method according to the invention faster and easier.

In certain embodiments, a DRE is performed prior to obtaining the urine sample. This can be beneficial in instances where a first IDO test according to the invention gave uninformative results, due to technical issues concerning the control and target gene.

In certain embodiments, IDO mRNA is quantified in said urine sample.

In certain embodiments, the quantification of IDO mRNA is effected by polymerase chain reaction (PCR).

In certain embodiments, the quantification of IDO mRNA is performed by quantitative real time polymerase chain reaction (qPCR).

In certain embodiments, the quantification of IDO mRNA is performed by digital polymerase chain reaction (dPCR). Digital PCR is a biotechnological refinement of conventional PCR methods that can be used to directly quantify and clonally amplify nucleic acids strands. Digital PCR improves upon the conventional PCR practices by dividing up the reaction into multiple, smaller reactions. A sample is partitioned so that individual nucleic acid molecules within the sample are localized within many separate regions. Micro well plates, capillaries, oil emulsion, and arrays of miniaturized chambers with nucleic acid binding surfaces can be used to partition the samples.

Alternatively, quantification of IDO mRNA may be effected by other techniques known to the person skilled in the art.

The skilled person is aware of suitable methods and formulae to calculate the ratio between IDO and control gene expression. An exemplary method is described in "Materials and Methods" below.

In certain embodiments, the control gene is any gene expressed by prostate cells at higher levels than IDO. The skilled person is aware that the expression of the control gene has to be independent of the presence of prostate cancer. In certain embodiments, the control gene is a housekeeping gene expressed by prostate cells at higher levels than IDO. In certain embodiments, the control gene is GAPDH.

In certain embodiments, a risk of having prostate cancer is excluded for the patient if the level of IDO mRNA is below a predetermined mRNA threshold 1. In other words, a patient's risk of having prostate cancer is determined to be (close to) 0 if the level of IDO mRNA is below the predetermined mRNA threshold 1.

In certain embodiments, the predetermined mRNA threshold 1 is an absolute copy number of IDO mRNA molecules between 0 and 100 copies within the sample. The sample can be a volume of non-processed urine, in particular between 0.1 ml and 5 ml, more particularly between 0,2 ml and 2 ml, more particularly between 0.25 and 1 ml, even more particularly approximately 0.5 ml. The sample can be a pellet obtained by centrifugation of urine, in particular by centrifugation of 2 ml to 100 ml of urine, more particularly 5 ml to 75 ml of urine, more particularly 15 ml to 50 ml of urine, even more particularly approximately 20 ml of urine.

In certain embodiments, the predetermined mRNA threshold 1 is a ratio of IDO mRNA copy number relative to the expression of a control gene.

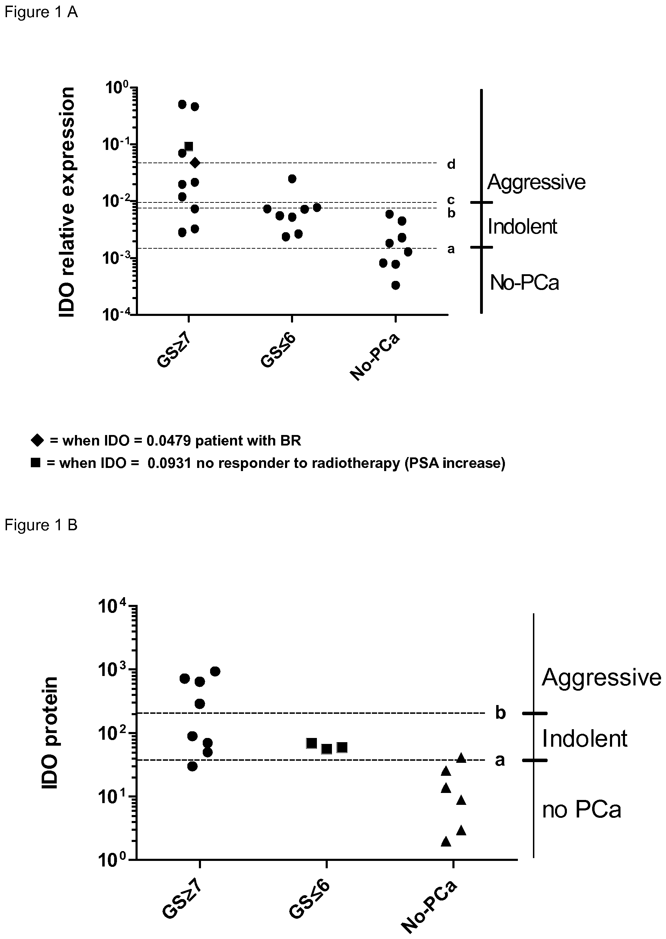

A patient with IDO mRNA levels below this mRNA threshold 1 has 0% or negligible probability of having PCa (Figure 1A), therefore a prostate biopsy is not recommended.

In certain embodiments, a risk of having or developing prostate cancer is assigned to the patient if the level of IDO mRNA is elevated in comparison to the IDO mRNA levels of a control population of individuals not suffering from prostate cancer. This is demonstrated by the results of the prostate biopsy or by the results of the prostatectomy (when available).

In certain embodiments, a significant risk of having prostate cancer is assigned to the patient if the level of IDO mRNA is above the predetermined mRNA threshold 1.

In certain embodiments, a risk of having prostate cancer (with a higher probability of having an indolent prostate cancer than a clinically relevant cancer) is assigned to the patient if the level of IDO mRNA is below a predetermined mRNA threshold 2, but above a predetermined mRNA threshold 1.

In certain embodiments, said predetermined mRNA threshold 2 is between 1.5 and 6 folds the predetermined mRNA threshold 1.

A patient with IDO mRNA levels below this predetermined mRNA threshold 2 but above a predetermined mRNA threshold 1 is assigned to the group for which a biopsy might be recommended (Figure 1A).

In certain embodiments, a higher risk of having prostate cancer (with a higher probability of having a clinically relevant cancer than an indolent prostate cancer) is assigned to the patient if the level of IDO mRNA is above a predetermined mRNA threshold 3.

In certain embodiments, said predetermined mRNA threshold 3 is between 6 and 10 folds the predetermined mRNA threshold 4.

A patient with IDO mRNA levels above this mRNA threshold 3 is assigned to the group for which a biopsy is always recommended.

In certain embodiments, a high risk of having a clinically relevant prostate cancer is assigned to the patient if the level of IDO mRNA is above the predetermined mRNA threshold 3. In certain embodiments, a high risk of having a prostate cancer characterized by a Gleason score ≥ 7 is assigned to the patient if the level of IDO mRNA is above the predetermined mRNA threshold 3. In certain embodiments, a high risk of having a prostate cancer that requires treatment after prostate biopsy is assigned to the patient if the level of IDO mRNA is above the predetermined mRNA threshold 3. For these patients the prostate biopsy is always recommended.

In certain embodiments, a high risk of biochemical recurrence of prostate cancer within 5 years is assigned to a patient if the level of IDO mRNA is above the predetermined mRNA threshold 3.

In certain embodiments, a high risk of having a clinically relevant prostate cancer is assigned to the patient if the level of IDO mRNA is above the predetermined mRNA threshold 4. In certain embodiments, a high risk of having a prostate cancer characterized by a Gleason score ≥ 7 is assigned to the patient if the level of IDO mRNA is above the predetermined mRNA threshold 1. In certain embodiments, a high risk of having a prostate cancer that requires treatment after prostate biopsy is assigned to the patient if the level of IDO mRNA is above the predetermined mRNA threshold 4. For these patients the prostate biopsy is always recommended.

In certain embodiments, a high risk of having a prostate cancer with higher chances of progression and metastatization is assigned to the patient if the level of IDO mRNA is above a predetermined mRNA threshold 4.

In certain embodiments, a risk of relapsing is assigned to the patient if the level of IDO mRNA is above a predetermined mRNA threshold 4.

In certain embodiments, a high risk of biochemical recurrence of prostate cancer within 5 years is assigned to a patient if the level of IDO mRNA is above the predetermined mRNA threshold 4.

In certain embodiments, the fourth predetermined mRNA threshold is more than 20 fold higher than mRNA threshold 1. A patient with IDO mRNA levels above this mRNA threshold 4 is assigned to the group for which a biopsy is always recommended for urgent treatment option (Figure 1A).

### IDO mRNA expression thresholds relative to GAPDH expression

In certain embodiments, the predetermined mRNA threshold 1 is between 0.0001 and 0.004 fold expression relative to GAPDH expression. In certain embodiments, the predetermined mRNA threshold 1 is **0.0015** fold IDO expression relative to GAPDH expression.

The inventors' data demonstrate that a patient with IDO mRNA levels below this mRNA threshold 1 has 0% or negligible probability of having PCa (Figure 1A).

In certain embodiments, the predetermined mRNA threshold 2 is between 0.004 and 0.0090 fold expression relative to GAPDH. In certain embodiments, the predetermined mRNA threshold 2 is **0.0075** fold IDO expression relative to GAPDH expression.

In certain embodiments, the predetermined mRNA threshold 3 is between 0.0090 and 0.03 fold expression relative to GAPDH. In certain embodiments, the predetermined mRNA threshold 3 is **0.0096** fold IDO expression relative to GAPDH expression.

The inventors' data demonstrate that 87.5% of patients with indolent PCa (GS ≤ 6 and BRand no treatment after biopsy), 100% of patients with no PCa and 27% of patients with clinically relevant PCa (GS ≥ 7, BR-, treatment after biopsy) expressed IDO below 0.0096 (Figure 1A).

The inventors' data demonstrate that 72% of patients with clinically relevant PCa (GS ≥ 7, treatment after biopsy) expressed IDO above 0.0096 (Figure 1A).

In certain embodiments, the predetermined mRNA threshold 4 is between 0.03 and 0.05 fold expression relative to GAPDH expression. In certain embodiments, the predetermined mRNA threshold 4 is **0.0479** fold IDO expression relative to GAPDH expression.

The inventors' data demonstrate that 100% of patients with BR+ PCa (GS ≥ 7, treatment after biopsy, BR within 5 years) or not responding to radiotherapy expressed IDO above 0.0479 (Figure 1A).

In certain embodiments, IDO protein is quantified in said urine sample. In certain embodiments, the quantification of IDO protein is performed by enzyme-linked immunosorbent assay (ELISA).

Alternatively, quantification of IDO protein may be effected by other techniques known to the person skilled in the art.

In certain embodiments, a risk of having prostate cancer is excluded for the patient if the level of IDO protein is below a predetermined protein threshold 1. In certain embodiments, a patient's risk of having prostate cancer is determined to be very low if the level of IDO protein is below the predetermined protein threshold 1. In certain embodiments, the predetermined protein threshold 1 is an absolute concentration of IDO protein, between 0 and 100 pg/ml.

A patient with IDO protein levels below this protein threshold 1 has low probability of having PCa (Figure 1B), therefore a prostate biopsy is not recommended.

In certain embodiments, a risk of having or developing prostate cancer is assigned to the patient if the level of IDO protein is elevated in comparison to the IDO protein levels of a control population of individuals not suffering from prostate cancer. This is demonstrated by the results of the prostate biopsy or by the results of the prostatectomy (when available).

In certain embodiments, a significant risk of having prostate cancer is assigned to the patient if the level of IDO protein is above a predetermined protein threshold 1.

In certain embodiments, a higher risk of having prostate cancer (with a higher probability of having an indolent prostate cancer than a clinically relevant cancer) is assigned to the patient if the level of IDO protein is above a predetermined protein threshold 2. In certain embodiments, said predetermined protein threshold 2 is between 1.5 and 6 folds the predetermined protein threshold 1. In certain embodiments, said predetermined protein threshold 2 is between 100 and 300 pg/ml. In certain embodiments, said predetermined protein threshold 2 is 200 pg/ml.

In certain embodiments, a patient with IDO protein levels below protein threshold 2 but above a protein threshold 1 is assigned to the group for which a biopsy can be recommended.

In certain embodiments, a patient with IDO protein levels above this protein threshold 2 is assigned to the group for which a biopsy is always recommended.

In certain embodiments, a higher risk of having prostate cancer (with a higher probability of having a clinically relevant cancer than an indolent prostate cancer) is assigned to the patient if the level of IDO protein is above a predetermined protein threshold 3. In certain embodiments, said predetermined protein threshold 3 is more than 300 pg/ml or more than double the predetermined protein threshold 1. For these patients the prostate biopsy is always recommended.

The inventors' data demonstrate that 83% of patients with no PCa have an IDO protein level below 200 pg. The inventors' data demonstrate that 87.5% of patients with clinically relevant PCa (GS ≥ 7, BR-, treatment after biopsy) have an IDO protein level above 200 pg. The inventors' data demonstrate that 100% of patients with indolent PCa or no PCa have an IDO protein level below protein threshold 3 (Figure 1B). In certain embodiments, the urine sample is not processed and allows for the analysis of RNA or protein.

In certain embodiments, the urine sample is a volume of urine with the addition of a reagent to preserve RNA.

In certain embodiments, the urine sample is a pellet obtained by centrifuging urine. In certain embodiments, the urine sample is a pellet obtained by centrifuging urine at above 300 g for > 5 min. In certain embodiments, the urine sample is a pellet obtained by centrifuging urine at between 200 and 2000 G for > 5 min. In certain embodiments, the urine sample is a pellet obtained by centrifuging urine at 300 g for > 5 min. In certain embodiments, the urine sample is a pellet obtained by centrifuging urine at > 700 g for 10 min or more.

In certain embodiments, the method comprises the following steps:
a. the urine sample is centrifuged immediately after collection for at least 5 min, particularly between 5 and 30 minutes, more particularly for between 8 and 15 min at below 10°C, particularly between 10°C and 4°C;
b. optionally, one or more RNAse inhibitor compounds are added to the sample at the time of collection or prior to centrifugation.

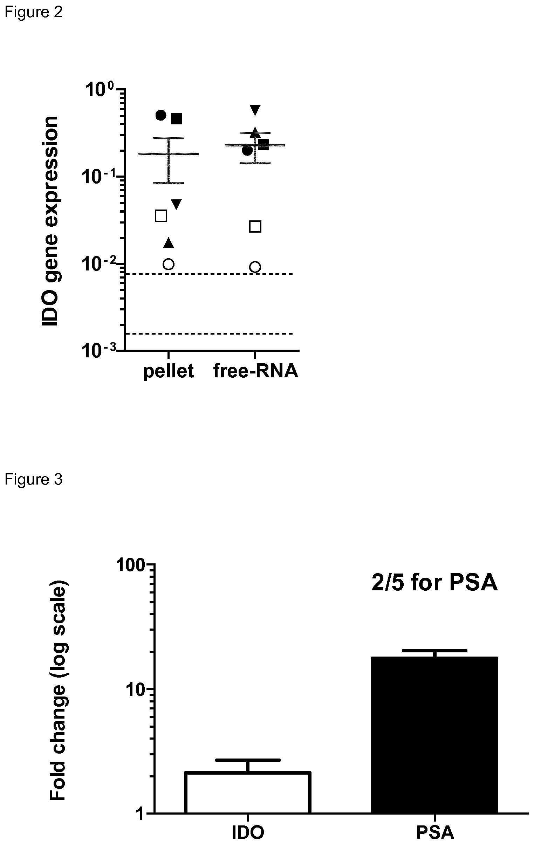

In certain embodiments, the urine sample is a cell free RNA sample. This expression relates to an RNA preparation obtained from a urine sample that has not been processed (e.g. by centrifugation) prior to RNA isolation. No differences between pellet and cell-free RNA in terms of IDO gene expression were detected (Fig. 2).

Urines should be processed immediately after collection, either by a centrifugation at 3°C - 25°C, in particular 4°C (in order to collect the pellet) or by aliquotation (for cell-free RNA or protein analysis). The addition of a solution to preserve RNA from degradation is also possible. It is also possible to keep the samples at 4°C after collection and perform centrifugation after several hours (e.g. 1-24 hours). To analyse cell-free RNA, RNA is extracted from 0.5 ml of urines without centrifugation. Optionally, one or more RNAse inhibitor or RNA stabilizer compounds are added to the sample at the time of collection. The IDO cDNA generated by retro-transcribing mRNA, is thus quantified by real time PCR by using a set of primers and probe (the TaqMan assay shown in the examples is one example thereof) targeting the IDO gene. One example of a suitable IDO region is the catalytic region. The TaqMan assay shown in the examples targets the catalytic region of IDO. The retrotranscription can be performed with target specific primers or with mixed primers. The retrotranscription can be a separate reaction or can be performed in the same reaction used for the quantification of the target sequence. The quantification can be performed by real time per or other quantitative methods, such as digital PCR or RNA-seq. A control gene is used to calculate the relative gene expression of IDO in order to make the test independent from PSA measurements.

In certain embodiments, the control gene is GAPDH. In certain embodiments, the 18s ribosomal RNA (rRNA) is used as internal control (technical control) to determine the amount of genetic material for the amplification. For 18s >7 Ct (threshold cycle in real-time PCR), the test might be uninformative and therefore a new urine sample must be collected and in order to repeat the test. In certain embodiments, DRE might be envisioned to improve the performance of the technical control gene.

In certain embodiments, the method employs at least one, and particularly all of, the following primers and probes:
Primer F 5'-GAAGACCCAAAGGAGTTTGC-3' (SEQ ID NO 01)
Primer R 5'-TGGAGGAACTGAGCAGCAT-3' (SEQ ID NO 02)
Probe : 5'-TGGGCATCCAGCAGACT-3' (SEQ ID NO 03).

In certain embodiments, the probe is modified with a fluorophore at the 5' or 3' end and a fluorophore quencher at the other end. The fluorophore quencher is capable of quenching the fluorescence emitted from the fluorophore upon excitation. For the specific probe sequence of SEQ ID NO 03, MGB is a particularly useful fluorophore quencher that can be used in combination with different fluorophores. Non-limiting examples are FAM/MGB, VIC/MGB, TWT/MGB, Yakima Yellow /MGB, HEX/MGB and Cy5/MGB. Other useful pairs of fluorophore and fluorophore quencher are carboxyfluorescein (FAM)/ (minor grove binder (MGB), FAM/black hole quencher 1 (BHQ1), YYE/BHQ1, ROX/BHQ2, Cy5/BHQ2.

In certain embodiments, the probe is modified with FAM (6-carboxy fluorescein) on the 5' and MGB on the 3' end.

In certain embodiments, IDO protein is quantified by ELISA. In certain embodiments, the quantification is performed by use of the IDK® IDO ELISA Kit by Immundiagnostik AG, Stubenwald-Allee 8a, 64625 Bensheim, Germany.

In certain embodiments, the urine sample is an unprocessed volume of urine. In the present specification, such a sample is also designated as "cell-free" RNA sample. "Unprocessed" relates to the fact that the sample has not been centrifuged prior to RNA isolation, therefore it comprises "total" urine, not a fraction separated from the primary sample (in other words: the sample as obtained from the patient) by centrifugation. The unprocessed RNA sample may comprise a solution that has been added to prevent RNA from degradation.

In certain embodiments, the urine sample is a pellet obtained by centrifuging a volume of urine.

IDO mRNA is expressed at high levels in PCa tissue. IDO protein has been reported to be present in PCa tissues by staining, but has never been quantified in an absolute manner. The observation that IDO mRNA and protein can be detected at high levels in urine of patients suspected of having prostate cancer, and its level can be used as a biomarker to discriminate tumour bearing patients or patients in need of further determination of their status by biopsy, is surprising:
The detection of mRNA and protein from a tissue affected by disease or other damage, into body fluids (such as blood, urines and saliva) differs depending on the type of damage suffered by the tissue during the development of the disease.

One case in point that may serve as reference is the detection of PSA. PSA is a highly specific prostate molecule, elevated levels of which in the peripheral circulation are an indicator of either prostate damage due to the presence of tumours, benign diseases or inflammation (i.e. prostatitis) or overproduction due to age or sexual intercourse. Notably, PSA is not tumour specific. PSA is currently the most commonly used biomarker for the diagnosis of PCa. PSA protein is secreted by prostatic cells into the lumen of prostatic grands and carried into prostatic duct. Thereby, it can reach and be detected in urines. PSA also physiologically enters the bloodstream in its inactive form (free PSA) at no-significantly levels (4 ng/ml), although the latter amount increases with age. During tumour development, more significantly at higher stage and grade, because of the damages to the prostatic gland structure, PSA protein levels significantly increase in the serum of patients and so far, only in this specimen it has a predictive power for prostate cancer. This secreted protein can be detected in urines, but its levels in urines alone are not predictive (Bolduc et al. (2007), Can. Urol. Assoc. J. 1, 377-81).

In homology, the fact the IDO is detectable in prostate cancer tissue at higher levels than in other conditions of the organ does not predict that it would be detectable in urines, much less that, in this specimen, it can be detected at more elevated levels in samples from patients having PCa tumours, compared to patients having other conditions. High levels of IDO have been detected in prostate tumor samples collected after prostatectomy, its presence in the prostate at earlier stages (for example in prostate biopsies) have never been reported, therefore the ability to detect IDO in the urines of patients at risk of harbouring prostate cancer (as indicated by PSA test) but whose diagnosis has yet to be assessed (before prostate biopsy), is unexpected. Furthermore, IDO is not a prostate-specific protein since other cells within the prostate can release it in specific areas where immunomodulation occurs. Therefore, other sources of IDO in the prostate, such as endothelial cells, macrophages etc., contribute to the background levels detectable in a patient. Relevant for a non-tumour-specific marker to be a valid diagnostic/prognostic tool is its significant overexpression over the physiological baseline in tumour bearing patients. Despite its background level, IDO is not physiologically abundantly secreted in urine and it does not significantly increase after prostatic massage, as PSA does (Figure 3). Therefore, its detection in this body fluid at elevated levels is not to be expected, as it is for PSA. Based on preliminary data, the inventors observed that IDO detection (mRNA and protein) in urine of patients at risk of PCa has a diagnostic power (Figure 1).

Urines, due to their physiological function, are specimen characterized by the presence of exfoliated cells, cell debris and discarded material, and are rich in RNases, DNases and other degrading enzymes. Therefore, they are considered a difficult material for the analysis of RNAs (considered unstable and easily and quickly degraded in such conditions), in comparison to blood, where cells can survive for longer periods. Furthermore, the presence of discarded material is expected to increase the background levels of molecules not exclusively expressed by one type of cells, such as IDO (protein is present in several type of cells in the prostate). The detection of IDO's RNA and protein in such specimen is therefore unexpected. Even more surprising is the possibility to use it as a quantitative marker with predictive powers (Figure 1).

### Terms and definitions

In the context of the present specification, the expression "mRNA" is meant to include any RNA species that can be used for the quantification of the expression of a given gene, including the primary transcript (also called pre-mRNA) and any intermediate construct formed during the processing of pre-mRNA to mRNA.

"PCa" in the context of the present specification relates to "prostate cancer".

"PSA" in the context of the present specification relates to "prostate specific antigen".

"RP" in the context of the present specification relates to "radical prostatectomy".

"RTx" in the context of the present specification relates to "radiation therapy".

"AS" in the context of the present specification relates to "active surveillance", a way of monitoring the evolution of a medical condition by regular testing, rather than immediate treatment such as surgery or chemotherapy.

"DRE" in the context of the present specification relates to "digital rectal examination".

"BR" in the context of the present specification relates to "biochemical recurrence" and specifies a rise in the detectable levels of a biochemical cancer marker, in particular a rise in the blood level of PSA. BR can occur in prostate cancer patients in which the prostate cancer has been treated, e.g. by surgery or radiation therapy. The affected patients may not exhibit symptoms. "BR-" means no biochemical recurrence of prostate cancer in a period of 5 years, "BR+" means biochemical recurrence of prostate cancer within 5 years.

"Relapse of PCa" in the context of the present specification is meant to include recurrence of PCa, in particular biochemical recurrence, and non-responsiveness/refractoriness to therapy.

"GS" in the context of the present specification relates to "Gleason Score". A Gleason score is a system of grading PCa tissue based on its microscopic appearance, evaluated by a pathologist. GS ranges from 2 to 10. The higher the GS, the more aggressive with a worse prognosis the PCa.

"PPV" in the context of the present specification relates to "positive predictive value".

"NPV" in the context of the present specification relates to "negative predictive value".

"GAPDH" in the context of the present specification relates to "Glyceraldehyde 3-phosphate dehydrogenase". GAPDH is constitutively expressed in most cells and can be used for the normalization of gene expression levels.

"Indolent prostate cancer" in the context of the present specification relates to a prostate cancer with a GS ≤ 6.

"Clinically relevant prostate cancer" in the context of the present specification relates to a prostate cancer that would be classified by the responsible physician as such. The diagnosis of a clinically relevant PCa results in a recommendation of treatment, usually by a method selected from surgery, chemotherapy, cryotherapy, hormonal therapy, and/or radiation therapy. In most instances, a clinically relevant PCa will be characterized by a GS ≥ 7.

The terms "symptomatic prostate cancer" and "aggressive prostate cancer" in the context of the present specification are used synonymously to "clinically relevant prostate cancer".

in the context of the present specification relates to a prostate cancer with a GS ≥ 7.

In the context of the present specification, the expression "risk (of having prostate cancer)" is synonymous with "likelihood (of having prostate cancer)".

In the context of the present specification, the expression "threshold" is synonymous to "cut-off". In the context of the present specification, the expression "threshold 1" is synonymous to "first threshold".

Wherever alternatives for single separable features are laid out herein as "embodiments", it is to be understood that such alternatives may be combined freely to form discrete embodiments of the invention disclosed herein. Thus, any of the alternative embodiments for a detectable label may be combined with any of the alternative embodiments of sequence and these combinations may be combined with any medical indication or diagnostic method mentioned herein.

The invention is further illustrated by the following examples and figures, from which further embodiments and advantages can be drawn. These examples are meant to illustrate the invention but not to limit its scope.

### Brief description of the figures

**Figure 1A** shows the definition of the thresholds for IDO mRNA and the feasibility of the test without performing DRE (prostate massage). Patients with GS ≥ 7 and GS ≤ 6 have PCa and the GS was evaluated from the biopsy or after prostatectomy, when available. GS ≥ 7 means clinically relevant PCa and GS ≤ 6 means indolent PCa. Cut-off 1 (a≤0.0015, assignment to biopsy or risk of having clinically relevant PCa: NPV 100%, PPV 47%; risk of having PCa: NPV = 100%, PPV = 82%), cut-off 2 (b≤0.0075, assignment to biopsy or risk of having clinically relevant PCa: NPV 81%, PPV 72%; risk of having PCa: NPV = 47%, PPV = 100%), cut-off 3 (c≥0.0096, assignment to biopsy or risk of having clinically relevant PCa: NPV 83%, PPV 88%) and cut-off 4 (d≥0.0479, risk of having BR within 5 years from treatment or treatment resistant-PCa: NPV 100%, PPV 40%). IDO relative gene expression was analysed in urine of patients collected without execution of a DRE.
   The IDO test reduces the number of unnecessary biopsies by: 14.8% with cut-off 1, or 51.8% with cut-off 2, or 66.6% with cut-off 3.
**Figure 1B** shows the thresholds for IDO protein and the feasibility of the test without performing DRE (prostate massage). Protein cut-off 1 (a ≤ 42 pg/ml, assignment to biopsy or risk of having clinically relevant PCa: PPV 83%, NPV 63%, risk of having PCa: PPV 90%, NPV 83%,), protein cut-off 2 (b≤ 200 pg/ml, , assignment to biopsy or risk of having clinically relevant PCa: PPV 69%, NPV 100%; risk of having PCa PPV 100%, NPV 85%) and the feasibility of the test without performing DRE (prostate massage). IDO relative gene expression was analysed in urine of patients collected without execution of a DRE. GS ≥ 7, GS ≤ 6 and no PCa are the results from the biopsy or prostatectomy. The IDO test reduces the number of unnecessary biopsies by 29.4 % with protein cut-off 2.
**Figure 2** shows the feasibility of the test in urine without centrifugation (free-RNA vs pellet).
**Figure 3****:** Prostatic massage increases the amount of mRNA PSA in urine by a factor of 50; (test performed in five patients comparing gene expression before and after DRE). Normally, DRE increases PSA protein by a factor of 3 in serum. Differently from PSA, the prostatic massage slightly increased the amount of IDO by 2 to 3-fold.

### Materials and Methods

### Collection of urines

Patient must restrain from any sexual activity in the previous 24 hours before collection. First urines of the morning are collected in a sterile container.

Pellet: 20 ml are centrifuged at 2000 rpm for 10 minutes, the supernatant is discarded and the pellet is used for RNA extraction.

Cell-free RNA and protein: 500 ul of urines are used for RNA extraction.

### RNA extraction

RNA extraction is performed according to the RNA Aqueous Kit protocol (Ambion). 700 ul of lysis solution is added either to the pellet or to 500 ul of urines. The elution step is performed twice in the same tube with a volume of 50 ul each time (final volume is 100 ul). Samples are stored at -80°C.

### Retro transcription

30ul of RNA are added to 30 ul of mix prepared with the High Capacity cDNA Reverse Transcription Kits (Applied Biosystems).

10ul of mix contains 2.0 µl of 10x RT Buffer, 0.8 ul of dNTPs, 2.0 ul of random primers, 1.0 ul of multiscribe reverse transcription enzyme and 4.2 ul of water. The reaction is performed in a thermocycler in 4 steps: 10 minutes at 25°C, 120 minutes at 37°C, 5 minutes at 85°C and 5 minutes at 4°C. Samples are stored at -80°C.

### Real-Time PCR

The IDO1 assay is designed covering the exon-exon junction between exon 9 and 10. The primers are designed in order to have a melting temperature between 58°C and 61°C, with an optimal length of 20 bp and CG content between 30% and 80%. The probe is designed in order to have a melting temperature 10°C higher than the one of the primers and does not start with a G. Primers and probe are used at a final concentration of 400nM and 200nM, respectively.

An exemplary TaqMan assay is represented by the sequences:
Primer F 5'-GAAGACCCAAAGGAGTTTGC-3' (SEQ ID NO 01)
Primer R 5'-TGGAGGAACTGAGCAGCAT-3' (SEQ ID NO 02)
Probe : 5'-TGGGCATCCAGCAGACT-3' (SEQ ID NO 03)

The probe is modified with FAM (6-carboxy fluorescein) on the 5 prime and a MGB (minor grove binder, non-fluorescent quencher fitting the FAM spectral qualities, purchased from Applied Biosystems) on the 3 prime terminus. The mix for the quantitative PCR is prepared using the TaqMan Gene Expression Master Mix (Applied Biosystems): it contains (per well) 10 ul of master mix 2x, 7 ul of water, 0.8 ul of primer F (10 uM), 0.8 ul of primer R (10 uM) and 0.4ul of probe (10 uM). 1ul of cDNA is tested per well. Samples are run in triplicate. For each run, a negative control is also tested in triplicate.

The PCR program is as follows: UNG incubation (2 minutes at 50°C); Polymerase activation (10 minutes at 95°C); PCR cycles (40X): denaturation (15 seconds at 95°C) and annealing (1 minute at 60°C).

### IDO's protein quantification

125 ul of urines were used for the quantification of IDO's protein by ELISA. The assay utilizes the two-site sandwich technique with two selected polyclonal antibodies that bind to human indoleamine 2,3-deoxygenase 1 (IDO1) (IDKR IDO ELISA kit K7727 by Immundiagnostik AG, Stubenwald-Allee 8a, 64625 Bensheim, Germany). The urine samples are added to the wells of a microplate coated with a high affine polyclonal anti-human IDO1 antibody. During the first incubation step IDO1 in the samples is captured by the immobilized antibody. Then, the biotinylated detection antibody, a polyclonal anti-human IDO1 antibody, is added. Peroxidase labeled streptavidin is added to the reaction and Tetramethylbenzidine (TMB) is used as a substrate for the peroxidase. An acidic stop solution is added to stop the reaction. A plate reader is used to measure the absorbance at 450nm and the concentration of IDO1 is calculated, using the values obtained from the standard curve.

### Data analysis

Normalization of gene expression was performed using either GAPDH or 18S. A 2^{-ΔΔCt} method was used to compute fold change of genes of interest (PSA and IDO) before and after DRE. A 2^{-ΔΔCt} method was also used to compute fold decrease of IDO as compared to GAPDH gene expression as 1. All value ≤10⁻⁷ were considered undetectable.

### Selection of specific cut-offs

By comparing patients diagnosed with clinically relevant PCa (GS ≥ 7), with indolent PCa (GS ≤ 6) and patients with PCa-free biopsies, specific cut-offs were selected.

Figure 1 illustrates the definition of **A)** cut-off 1 (0.0015), cut-off 2 (0.0075), cut-off 3 (0.0096), cut-off 4 (0.0479), **B)** cut-off 5 (42 pg/ml), cut-off 6 (200 pg/ml).

In order to identify patients undergoing biopsy with negative results (NPV 100%; 0% false positive), a first cut-off (cut-off 1) of 0.0015was generated.

In order to gather low risk PCa, a second cut-off (cut-off 2) of 0.0075 was generated. 82% of patients with PCa GS≤6 and BR- or no PCa expressed IDO<0.0075. For patients below this cut-off 2 a biopsy might not be recommended.

In order to gather intermediate/high risk PCa, a third cut-off (cut-off 3) of 0.0096 was generated. 93.7% of patients with PCa GS≤6 and BR- or no PCa expressed IDO<0.0096. For patients above this cut-off 3 a biopsy is always recommended.

In order to diagnose patients with a high probability to progress, a forth cut-off (cut-off 4) of 0.0479 was generated. Patients above this cut-off 4 should undergo immediate biopsy and probably treatment.

In order to identify patients undergoing biopsy with negative results (risk of PCa: PPV = 90%; NPV = 83%), a first protein cut-off (protein cut-off 1) of 42 pg/ml was generated.

In order to gather intermediate/high risk PCa, a second protein cut-off (protein cut-off 2) of 200 pg/ml was generated (PPV = 100%, NPV = 69%). For patients above this protein cut-off 2 a biopsy is always recommended.

## Claims

1. A method to
- predict a patient's risk of having or developing prostate cancer (PCa),
- assign a patient to prostate biopsy,
- predict a patient's risk of having clinically relevant PCa,
- predict a patient's risk of having indolent PCa,
- distinguish indolent vs. aggressive cancers,
- predict a patient's risk of relapse or biochemical recurrence of prostate cancer, and/or
- predict a patient's risk of progression of an indolent prostate cancer to clinically relevant prostate cancer;
wherein said method comprises detecting / quantifying the level of indoleamine-2,3-dioxygenase (IDO) mRNA or protein in a urine sample obtained from said patient.

2. The method according to claim 1, wherein no digital rectal examination (DRE) is performed prior to obtaining said urine sample.

3. The method according to any one of the above claims, wherein a risk of having prostate cancer is excluded for said patient if said level of IDO mRNA is below a predetermined mRNA threshold 1.

4. The method according to claim 3, wherein a risk of having prostate cancer is assigned to said patient if said level of IDO mRNA is below a predetermined mRNA threshold 2, but above said predetermined mRNA threshold 1.

5. The method according to any one of the above claims, wherein said patient is assigned to prostate biopsy if said level of IDO mRNA is above a predetermined mRNA threshold 3.

6. The method according to claim 1, wherein a high risk of having a prostate cancer with a Gleason score ≥ 7 is assigned to said patient if said level of IDO mRNA is above a predetermined threshold 3.

7. The method according to claim 1, wherein a high risk of biochemical recurrence of prostate cancer within 5 years is assigned to a patient having undergone prostate cancer treatment if said level of IDO mRNA is above said predetermined threshold 3.

8. The method according to any one of the above claims, wherein a risk of relapse is assigned to said patient if said level of IDO mRNA is above a predetermined mRNA threshold 4.

9. The method according to any one of the preceding claims, wherein quantifying IDO mRNA is performed by polymerase chain reaction (PCR), in particular quantitative real-time PCR (qPCR).

10. The method according to claim 1 or 2, wherein a low risk of having a prostate cancer is assigned to said patient if the level of IDO protein is below a predetermined protein threshold 1.

11. The method according to claim 1 or 2, wherein a definite risk of clinically relevant prostate cancer is assigned to a patient if the level of IDO protein is above a predetermined protein threshold 2.

12. The method according to any one of claims 1 to 2 or 10 to 11, wherein quantifying IDO protein is effected by enzyme-linked immunosorbent assay (ELISA).

13. The method according to any one of the above claims, wherein said urine sample is an unprocessed volume of urine.

14. The method according to any one of the above claims, wherein said urine sample is a pellet obtained by centrifuging a volume of urine.

## Patentansprüche

1. Ein Verfahren zum
- Vorhersagen von einem Risiko eines Patienten, Prostatakrebs (PCa) zu haben oder zu entwickeln
- Zuweisen eines Patienten zur Prostatabiopsie,
- Vorhersagen von einem Risiko eines Patienten, einen klinisch relevanten PCa zu haben,
- Vorhersagen von einem Risiko eines Patienten, indolenten PCa zu haben,
- Unterscheiden zwischen indolenten und aggressiven Krebsarten,
- Vorhersagen von einem Wiedererkrankungsrisiko eines Patienten oder biochemischem Wiederauftreten von Prostatakrebs, und/oder
- Vorhersagen von einem Progressionsrisiko eines Patienten von einem indolenten Prostatakrebs zu klinisch relevantem Prostatakrebs;
wobei besagtes Verfahren Nachweisen/Quantifizieren von dem Spiegel von Indolamin-2,3-Dioxygenase (IDO)-mRNA oder -Protein in einer von besagtem Patienten erhaltenen Urinprobe umfasst.

2. Das Verfahren gemäß Anspruch 1, wobei vor dem Erhalten besagter Urinprobe keine digitale rektale Untersuchung (DRE) durchgeführt wird.

3. Das Verfahren gemäß einem der obigen Ansprüche, wobei ein Risiko, Prostatakrebs zu haben, für besagten Patienten ausgeschlossen ist, wenn besagter Spiegel von IDO-mRNA unter einem vorbestimmten mRNA-Schwellenwert 1 ist.

4. Das Verfahren gemäß Anspruch 3, wobei ein Risiko, Prostatakrebs zu haben, besagtem Patienten zugewiesen wird, wenn besagter Spiegel von IDO-mRNA unter einem vorbestimmten mRNA-Schwellenwert 2, aber über besagtem vorbestimmten mRNA-Schwellenwert 1 ist.

5. Das Verfahren gemäß einem der obigen Ansprüche, wobei besagter Patient einer Prostatabiopsie zugewiesen wird, wenn besagter Spiegel von IDO-mRNA über einem vorbestimmten mRNA-Schwellenwert 3 ist.

6. Das Verfahren gemäß Anspruch 1, wobei ein hohes Risiko Prostatakrebs mit einem Gleason-Score ≥ 7 zu haben, besagtem Patienten zugewiesen wird, wenn besagter Spiegel von IDO-mRNA über einem vorbestimmten Schwellenwert 3 ist.

7. Das Verfahren gemäß Anspruch 1, wobei ein hohes Risiko von biochemischem Wiederauftreten von Prostatakrebs innerhalb von 5 Jahren einem Patienten zugewiesen wird, der sich einer Prostatakrebsbehandlung unterzogen hat, wenn besagter Spiegel von IDO-mRNA über besagtem vorbestimmten Schwellenwert 3 ist.

8. Das Verfahren gemäß einem der obigen Ansprüche, wobei besagtem Patienten ein Wiedererkrankungsrisiko zugewiesen wird, wenn besagter Spiegel von IDO-mRNA über einem vorbestimmten mRNA-Schwellenwert liegt 4.

9. Das Verfahren gemäß einem der vorstehenden Ansprüche, wobei Quantifizierung von IDO-mRNA durch Polymerase-Kettenreaktion (PCR), insbesondere quantitative Echtzeit-PCR (qPCR), durchgeführt wird.

10. Das Verfahren gemäß Anspruch 1 oder 2, wobei besagtem Patienten ein geringes Risiko, einen Prostatakrebs zu haben, zugewiesen wird, wenn besagter Spiegel von IDO-Protein unter einem vorbestimmten Proteinschwellenwert 1 ist.

11. Das Verfahren gemäß Anspruch 1 oder 2, wobei einem Patienten ein definitives Risiko für klinisch relevanten Prostatakrebs zugewiesen wird, wenn der Spiegel von IDO-Protein über einem vorbestimmten Proteinschwellenwert 2 ist.

12. Das Verfahren gemäß einem der Ansprüche 1 bis 2 oder 10 bis 11, wobei Quantifizierung von IDO-Protein durch einen enzyme-linked immunosorbent assay (ELISA) durchgeführt wird.

13. Das Verfahren gemäß einem der obigen Ansprüche, wobei besagte Urinprobe ein unbehandeltes Urinvolumen ist.

14. Das Verfahren gemäß einem der obigen Ansprüche, wobei besagte Urinprobe ein Pellet ist, das durch Zentrifugieren von einem Urinvolumen erhalten wird.

## Revendications

1. Procédé pour
- prédire le risque de développement d'un cancer de la prostate (PCa) pour un patient,
- attribuer une biopsie de la prostate à un patient,
- prévoir le risque d'un PCa cliniquement pertinent pour un patient,
- prédire le risque d'un PCa indolent pour un patient,
- distinguer les cancers indolents des cancers agressifs,
- prédire le risque de rechute ou de récidive biochimique d'un patient du cancer de la prostate, et/ou
- prédire le risque de progression d'un cancer de la prostate indolent vers un cancer de la prostate cliniquement pertinent pour un patient ;
dans lequel ledit procédé comprend la détection/quantification du niveau d'ARNm ou de protéine d'indoleamine-2,3-dioxygénase (IDO) ou dans un échantillon d'urine obtenu à partir dudit patient.

2. Procédé selon la revendication 1, dans lequel aucun toucher rectal (DRE) n'est effectué avant l'obtention dudit échantillon d'urine.

3. Procédé selon l'une quelconque des revendications précédentes, dans lequel un risque de cancer de la prostate est exclu pour ledit patient si ledit niveau d'ARNm d'IDO est inférieur à un seuil 1 d'ARNm prédéterminé.

4. Procédé selon la revendication 3, dans lequel un risque de cancer de la prostate est attribué audit patient si ledit niveau d'ARNm d'IDO est inférieur à un seuil 2 prédéterminé d'ARNm, mais supérieur audit seuil 1 prédéterminé d'ARNm.

5. Procédé selon l'une quelconque des revendications précédentes, dans lequel ledit patient se voit attribuer une biopsie de la prostate si ledit niveau d'ARNm d'IDO est supérieur à un seuil 3 d'ARNm prédéterminé.

6. Procédé selon la revendication 1, dans lequel un risque élevé d'avoir un cancer de la prostate avec un score de Gleason ≥ 7 est attribué audit patient si ledit niveau d'ARNm d'IDO est supérieur à un seuil 3 prédéterminé.

7. Procédé selon la revendication 1, dans lequel un risque élevé de récidive biochimique du cancer de la prostate dans les 5 ans est attribué à un patient ayant reçu un traitement contre le cancer de la prostate si ledit niveau d'ARNm d'IDO est supérieur audit seuil 3 prédéterminé.

8. Procédé selon l'une quelconque des revendications précédentes, dans lequel un risque de rechute est attribué audit patient si ledit niveau d'ARNm d'IDO est supérieur à un seuil 4 d'ARNm prédéterminé.

9. Procédé selon l'une quelconque des revendications précédentes, dans lequel la quantification d'ARNm d'IDO est effectuée par amplification en chaîne par polymérase (PCR), en particulier par PCR quantitative en temps réel (qPCR).

10. Procédé selon la revendication 1 ou 2, dans lequel un faible risque d'avoir un cancer de la prostate est attribué audit patient si le niveau de protéine IDO est inférieur à un seuil 1 de protéine prédéterminé.

11. Procédé selon la revendication 1 ou 2, dans lequel un risque défini de cancer de la prostate cliniquement pertinent est attribué à un patient si le niveau de protéine IDO est supérieur à un seuil 2 de protéine prédéterminé.

12. Procédé selon l'une quelconque des revendications 1 à 2 ou 10 à 11, dans lequel la quantification de la protéine IDO est effectuée par dosage immunoenzymatique (ELISA).

13. Procédé selon l'une quelconque des revendications précédentes, dans lequel ledit échantillon d'urine est un volume d'urine non traité.

14. Procédé selon l'une quelconque des revendications précédentes, dans lequel ledit échantillon d'urine est une pastille obtenue par centrifugation d'un volume d'urine.
